Europäisches Patentamt

European Patent Office

Office européen des brevets

(19)

(11)  **EP 1 095 276 B1**

## (12)  FASCICULE DE BREVET EUROPEEN

(45) Date de publication et mention
de la délivrance du brevet:
**06.03.2002  Bulletin 2002/10**

(21) Numéro de dépôt: **99929407.7**

(22) Date de dépôt: **05.07.1999**

(51) Int Cl.$^7$: **G01N 33/574**

(86) Numéro de dépôt international:
**PCT/FR99/01622**

(87) Numéro de publication internationale:
**WO 00/02052 (13.01.2000 Gazette 2000/02)**

(54) **METHODE DE DIAGNOSTIC D'UN ADENOCARCINOME OU D'UNE PATHOLOGIE BENIGNE DE LA PROSTATE**

VERFAHREN ZUR DIAGNOSE ADENOKARZINOM ODER BENIGNE PATHOLOGIE DER VORSTEHERDRÜSE

METHOD FOR DIAGNOSING AN ADENOCARCINOMA OR A BENIGN PROSTATE PATHOLOGY

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité:  **03.07.1998  FR 9808702**

(43) Date de publication de la demande:
**02.05.2001  Bulletin 2001/18**

(73) Titulaire: **BIO MERIEUX**
**69280 Marcy l'Etoile (FR)**

(72) Inventeurs:
• **CHARRIER, Jean-Philippe**
**F-69130 Ecully (FR)**
• **TOURNEL, Carole**
**F-69380 Chasselay (FR)**
• **JOLIVET, Michel**
**F-69500 Bron (FR)**

(74) Mandataire: **Guerre, Dominique et al**
**Cabinet Germain et Maureau, 12, rue Boileau, BP 6153**
**69466 Lyon Cedex 06 (FR)**

(56) Documents cités:
**WO-A-97/12245**

• **BIOLOGICAL ABSTRACTS, Philadelphia PA USA; abstract no. PREV199799572557, * abrégé * XP002112544 & Z. CHEN ET AL.: "Prostate specific antigen in benign prostatic hyperplasia: purification and characterization" JOURNAL OF UROLOGY, vol. 157, no. 6, 1997, pages 2166-2170, Stanford CA USA**
• **MEDLINE, Washington DC USA; abstract no. 99274084, abrégé XP002112347 & J.P CHARRIER ET AL.: "Two-dimentional electrophoresis of prostate-specific antigen in sera of men with prostate cancer or benign prostate hyperplasia" ELECTROPHORESIS, vol. 20, no. 4-5, 1 avril 1999 (1999-04-01), pages 1075-1081, FRG**

EP 1 095 276 B1

**Description**

La présente invention concerne une méthode de dépistage ou de diagnostic permettant de mettre en évidence la présence d'un cancer de la prostate ou d'une hypertrophie bénigne de la prostate (BPH) chez un patient, et ceci sans réaliser de biopsie.

*En effet, l'antigène spécifique de la prostate, plus communément appelé PSA, est le principal marqueur du cancer de la prostate qui affectera, au cours de sa vie, un homme sur six en occident. Cette protéase de la famille des kallikréines, principalement sécrétée par l'épithélium prostatique, est retrouvée à une concentration de 0,5 à 5 mg/ml dans le liquide séminal et à une concentration un million de fois moindre dans le sérum d'un patient. Ce taux de PSA sérique augmente notablement lors d'un cancer de la prostate et modérément lors d'altérations bénignes, telles que BPH, prostatite aiguë...*

*Cependant, la zone de recouvrement entre les différentes pathologies est responsable d'un manque important de sensibilité et de spécificité. C'est ainsi que 30 à 45 % des cancers confinés à la glande, qui constitue un stade précoce et potentiellement curable, ne sont pas dépistés avec le seuil usuel de 4 ng/ml alors que trois patients sur quatre sont suspectés à tort.*

*Par ailleurs, il a été montré récemment que dans le sérum, le PSA s'associait à des inhibiteurs de protéase, tels que l'α-1 antichymostrypsine (ACT), et que l'usage du ratio PSA libre sur PSA total permettait d'améliorer la spécificité du diagnostic.*

*L'art antérieur montre donc qu'il existe des techniques permettant de diagnostiquer le développement d'un cancer de la prostate chez les patients. Ainsi, la demande de brevet WO-A-97/12245 revendique une méthode permettant de diagnostiquer un adénocarcinome de la prostate (CAP) sans biopsie. Cette méthode consiste à mesurer, dans le sérum ou dans le sang des patients, la quantité totale de PSA. Si cette valeur est comprise entre 2,5 et 20 ng/ml, on mesure également la concentration de PSA libre. On calcule ensuite le ratio PSA libre sur PSA total. Si ce ratio est inférieur à 7 %, le diagnostic s'oriente vers un adénocarcinome de la prostate.*

Toutefois, l'utilisation d'un seuil à 7 %, pour le diagnostic d'un cancer de la prostate, est controversée par de nombreux auteurs, comme le montre la publication de Lein et al. « Relation of free PSA/total PSA in serum for differentiating between patients with prostatic cancer and benign prostate hyperplasia : which cutoff should be used ?». Dans ce document publié dans la revue Cancer Investigation, 16(1), 45-49, 1998, il a été démontré qu'il est difficile, par le biais de ce ratio, de différencier systématiquement un cancer ou adénocarcinome de la prostate, d'une BPH.

En outre la publication de Catalona et al., " Prostate Cancer Detection in Men With Serum PSA Concentrations of 2.6 to 4.0 ng/ml and Benign Prostate Examination ", publiée dans JAMA du 14 Mai 1997-Vol 277, No.18, démontre qu'une concentration inférieure à 4 ng/ml doit être prise en considération pour dépister précocement un cancer de la prostate.

Par ailleurs, dans le liquide séminal, le PSA peut présenter des sites de clivages internes qui n'affectent pas sa cohésion tridimensionnelle du fait de l'existence de cinq ponts Bisulfures qui lient les différents fragments de la molécule. Le PSA ainsi clivé perd son activité enzymatique et sa capacité à s'associer aux inhibiteurs de protéase. Mais dans le sérum, aucune forme clivée n'est généralement mentionnée dans l'état de la technique.

Aucune forme clivée n'a jusqu'à ce jour été rapportée en cas de pathologies bénignes de la prostate et l'intérêt des formes clivées n'a pas été exploré à des fins de diagnostic.

Conformément à la présente invention, la méthode de diagnostic proposée permet de combler le manque d'efficacité des tests dans la zone de concentration de PSA totale supérieur à 2 ng/ml. A cette fin, les formes moléculaires de PSA sérique de patients atteints de cancer ou de BPH ont été cartographiées par électrophorèse bi-dimensionnelle, associé à la détection par chimiluminescence, afin d'observer l'ensemble des formes de PSA libre, complexé et clivé.

Ainsi, les profils de sérum de cancéreux sont relativement homogènes, alors que ceux de BPH peuvent comporter une proportion relativement importante de formes clivées, et des spots légèrement plus basiques.

Il est donc démontré que l'augmentation du ratio PSA libre sur PSA total en cas de BPH peut être lié à l'existence de PSA clivé, enzymatiquement inactif et incapable de se lier à l'ACT, ou être en relation avec du PSA libre légèrement basique qui pourrait correspondre à la forme zymogène inactive.

A cet effet, la présente invention concerne une première méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, caractérisée en ce qu'elle consiste à :

- mesurer le niveau de PSA libre total, c'est-à-dire clivé ou non clivé,
- mesurer le niveau de tout ou partie du PSA libre clivé,
- calculer la proportion de PSA libre clivé par rapport au PSA libre total, et
- distinguer deux cas, à savoir :

- ce rapport a une valeur inférieure ou égale à une valeur de référence ou de différenciation, trouvée chez des patients affectés par un adénocarcinome de la prostate, de sorte que le patient est atteint d'un adénocarcinome de la prostate,
- ce rapport a une valeur supérieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

La présente invention concerne une deuxième méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, caractérisée en ce qu'elle consiste à :

- mesurer le niveau de PSA libre non clivé,
- mesurer le niveau de tout ou partie du PSA libre clivé,
- calculer la proportion de PSA libre clivé par rapport au PSA libre non clivé, et
- distinguer deux cas, à savoir :

- ce rapport a une valeur inférieure ou égale à une valeur de référence ou de différenciation, trouvée chez des patients affectés par un adénocarcinome de la prostate, de sorte que le patient est atteint d'un adénocarcinome de la prostate,
- ce rapport a une valeur supérieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

Dans ces deux premiers cas, la valeur de référence est comprise entre 2 et 12 % et préférentiellement entre 5 et 8 %.

La présente invention concerne une troisième méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, caractérisée en ce qu'elle consiste à :

- mesurer le niveau de PSA libre total, c'est-à-dire clivé ou non clivé,
- mesurer le niveau de tout ou partie du PSA libre non clivé,
- calculer la proportion de PSA libre non clivé par rapport au PSA libre total, et
- distinguer deux cas, à savoir :

- ce rapport a une valeur supérieure ou égale à une valeur de référence ou de différenciation, trouvée chez des patients affectés par un adénocarcinome de la prostate, de sorte que le patient est atteint d'un adénocarcinome de la prostate,
- ce rapport a une valeur inférieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

Dans ce troisième cas, la valeur de référence est comprise entre 88 et 98 % et préférentiellement entre 93 et 95 %.

La présente invention concerne une quatrième méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, caractérisée en ce qu'elle consiste à :

- mesurer le niveau de PSA total, c'est-à-dire complexé et libre (clivé ou non clivé),
- mesurer le niveau de tout ou partie du PSA libre clivé,
- calculer la proportion de PSA libre clivé par rapport au PSA total, et
- distinguer deux cas, à savoir :

- ce rapport a une valeur inférieure ou égale à une valeur de référence ou de différenciation, trouvée chez des patients affectés par un adénocarcinome de la prostate, de sorte que le patient est atteint d'un adénocarcinome de la prostate,

- ce rapport a une valeur supérieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

Dans ce quatrième cas, la valeur de référence est comprise entre 0,1 et 2 % et préférentiellement entre 0,4 et 1 %.

La présente invention concerne une première méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, caractérisée en ce qu'elle consiste à :

- mesurer le niveau de PSA complexé,
- mesurer le niveau de tout ou partie du PSA libre clivé,
- calculer la proportion de PSA libre clivé par rapport au PSA complexé, et
- distinguer deux cas, à savoir :

  - ce rapport a une valeur supérieure ou égale à une valeur de référence ou de différenciation, trouvée chez des patients affectés par un adénocarcinome de la prostate, de sorte que le patient est atteint d'un adénocarcinome de la prostate,
  - ce rapport a une valeur inférieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

Dans ce cinquième cas, la valeur de référence est comprise entre 0,1 et 2,2 % et préférentiellement entre 0,6 et 1,2 %.

La présente invention concerne une sixième méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, caractérisée en ce qu'elle consiste à :

- mesurer le niveau de PSA libre non clivé,
- mesurer le niveau de tout ou partie du PSA libre clivé,
- mesurer le niveau de PSA libre,
- mesurer le niveau de PSA total,
- calculer la proportion de PSA libre clivé par rapport au PSA libre non clivé, dit premier rapport,
- calculer la proportion de PSA libre par rapport au PSA total, dit second rapport, et
- distinguer quatre cas, à savoir :

  - ce premier rapport a une valeur inférieure ou égale à une première valeur de référence ou de différenciation et ce second rapport a une valeur inférieure ou égale à une seconde valeur de référence ou de différenciation, de sorte que le patient est atteint d'un adénocarcinome de la prostate,
  - ce premier rapport a une valeur supérieure ou égale à une première valeur de référence ou de différenciation et ce second rapport a une valeur supérieure ou égale à une seconde valeur de référence ou de différenciation, de sorte que le patient est atteint d'une pathologie bénigne de la prostate, telle qu'une hypertrophie bénigne de la prostate,
  - ce premier rapport a une valeur inférieure ou égale à une première valeur de référence ou de différenciation et ce second rapport a une valeur supérieure ou égale à une seconde valeur de référence ou de différenciation, de sorte que le patient a une forte probabilité d'être atteint d'un adénocarcinome de la prostate et une faible probabilité d'être atteint d'une pathologie bénigne de la prostate, telle qu'une hypertrophie bénigne de la prostate,
  - ce premier rapport a une valeur supérieure ou égale à une première valeur de référence ou de différenciation et ce second rapport a une valeur inférieure ou égale à une seconde valeur de référence ou de différenciation, de sorte que le patient a une forte probabilité d'être atteint d'une pathologie bénigne de la prostate, telle qu'une hypertrophie bénigne de la prostate, et une faible probabilité d'être atteint d'un adénocarcinome de la prostate.

Dans ce sixième cas, la première valeur de référence est comprise entre 1 et 18 %, préférentiellement entre 5 et 8 % et plus précisément de sensiblement 6,3 %, et la seconde valeur de référence est comprise entre 10 et 20 %, préférentiellement entre 13 et 17 % et plus précisément de sensiblement 15 %.

Préférentiellement, dans le cadre de ce sixième cas, la forte probabilité d'être atteint d'un adénocarcinome de la prostate est comprise entre 80 et 95 % et préférentiellement entre 84 et 87 % et la faible probabilité d'être atteint d'une pathologie bénigne de la prostate est comprise entre 5 et 20 % et préférentiellement entre 13 et 16 %, alors que la forte probabilité d'être atteint d'une pathologie bénigne de la prostate est comprise entre 80 et 95 % et préférentiellement entre 82 et 85 %, et la faible probabilité d'être atteint d'un adénocarcinome de la prostate est comprise entre 5 et 20 % et préférentiellement entre 15 et 18%.

La présente invention concerne également une méthode d'analyse en vue du diagnostic d'un adénocarcinome ou d'une pathologie bénigne de la prostate chez un patient, qui utilise la combinaison d'au moins deux méthodes décrites ci-dessus.

Selon une variante de réalisation, elle est mise en oeuvre lorsque la concentration en PSA total dans le sang ou dans le sérum est supérieure à 2 ng/ml.

La présente invention concerne enfin un procédé utilisant une des méthodes qui viennent d'être décrites, il consiste à :

- détecter la présence et quantifier le niveau de deux des paramètres suivants : PSA libre sous forme clivée, PSA libre sous forme non clivée, PSA complexé et PSA total,
- déterminer la valeur de la proportion de :

  - PSA libre clivé par rapport au PSA libre total,
  - PSA libre clivé par rapport au PSA libre non clivé,
  - PSA libre non clivé par rapport au PSA libre total,
  - PSA libre clivé par rapport au PSA total, ou
  - PSA libre clivé par rapport au PSA complexé,

  et
- comparer cette proportion par rapport à une valeur de référence, en vue du diagnostic d'un adénocarcinome de la prostate chez un patient humain mâle.

Préalablement et préférentiellement on fait migrer les protéines issues du sang, du sérum, l'urine ou du liquide séminal du patient.

Préférentiellement, la migration s'effectue par électrophorèse bi-dimensionnelle.

Préalablement on dose le taux de PSA total.

Enfin et préférentiellement, la détection s'effectue par chimiluminescence après révélation par Western Blot.

Les figures ci-jointes sont données à titre d'exemple explicatif et n'ont aucun caractère limitatif. Elles permettront de mieux comprendre l'invention.

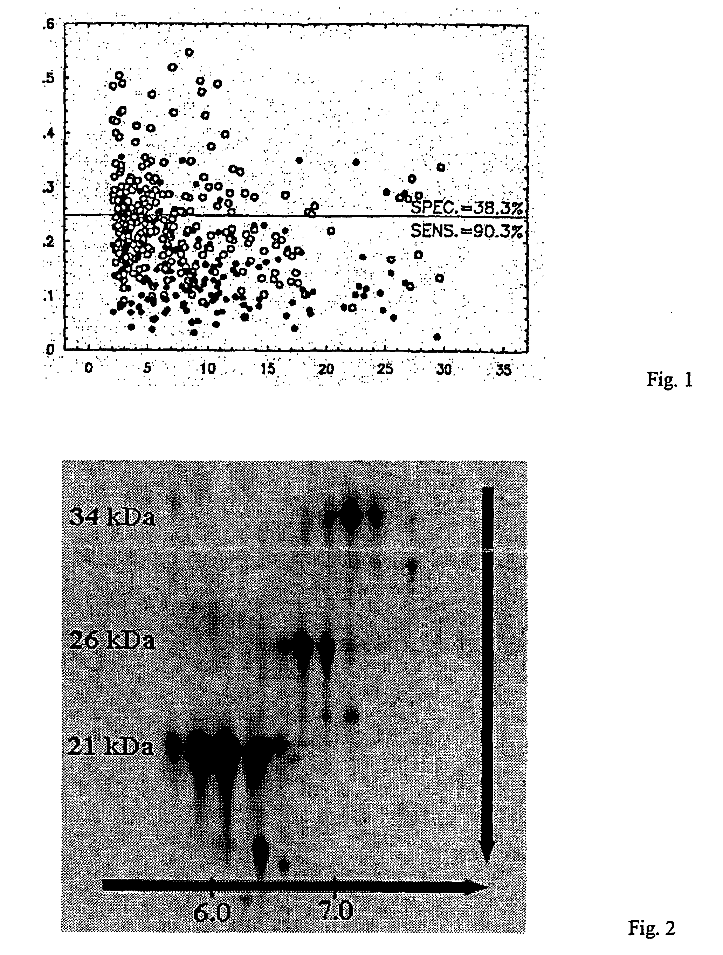

La figure 1 représente un tableau comparatif du positionnement de sérums de différents patients en fonction, d'une part, de leur concentration en PSA total, compris entre 2,1 et 30 ng/ml (axe des abscisses), et, d'autre part, de leur ratio PSA libre sur PSA total (axe des ordonnées). Ce tableau est tiré d'un article « Free to total Prostate-Specific Antigen (PSA) ratio is superior to total-PSA in differentiating Benign Prostate Hypertrophy from prostate cancer», Vol. 156, 1964-1968, dec 1996 selon P. J. Van Cangh et al. Les ronds noirs représentent les cas de cancer et les ronds blancs les cas de BPH.

La figure 2 représente une électrophorèse bi-dimensionnelle, où la migration de PSA, sous différentes formes, s'effectue en fonction, d'une part, du point isoélectrique en abscisse, et d'autre part, du poids moléculaire en ordonnée.

La figure 3 représente une électrophorèse bi-dimensionnelle d'un sérum d'un patient atteint d'une BPH, le sérum contenant 3,9 ng/ml de PSA.

La figure 4 représente une électrophorèse bi-dimensionnelle d'un sérum d'un patient atteint d'un adénocarcinome, le sérum contenant 4000 ng/ml de PSA.

La figure 5 représente un tableau comparatif du positionnement de sérums de différents patients en fonction, d'une part, de leur concentration en PSA total, compris entre 1 et 34 ng/ml (axe des abscisses), et, d'autre part, de leur ratio concentration PSA libre clivé sur concentration PSA libre non clivé (axe des ordonnées). Les carrés blancs représentent les cas de cancer et les ronds noirs les cas de BPH.

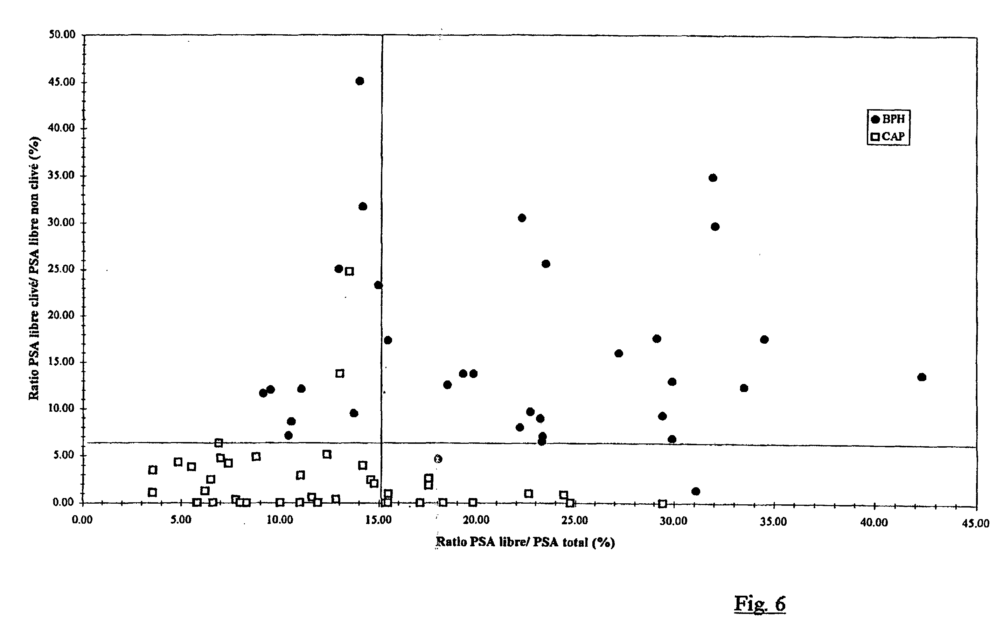

Enfin, la figure 6 représente un tableau comparatif du positionnement de sérums de différents patients en fonction, d'une part, de leur ratio concentration en PSA libre sur concentration en PSA total (axe des abscisses), et, d'autre part, de leur ratio concentration en PSA libre clivé sur PSA libre non clivé (axe des ordonnées). Les carrés blancs représentent les cas de cancer et les ronds noirs les cas de BPH.

La présente invention concerne une méthode de diagnostic du cancer de la prostate ou de pathologie bénigne de la prostate, telle que BPH, grâce au traitement de 100 μl d'échantillon de la façon suivante :

- ajout de 50 µl de solution en eau de Sulfate Dodecyl de Sodium (SDS) à 10 % et de Dithiothreitol (DTT) à 2,3 % ;
- chauffage pendant 5 minutes dans un bain marie porté à ébullition ;
- dilution dans QSP 350 µl de solution en eau d'urée 8,3 M, Thiourée 2 M, sulfonate de 3-[(3-cholamidopropyl)-diméthylammonio]-1-propane (CHAPS) 4 %, DTT 100 mM, Servalyte 4-9 (Serva, Heidelberg, Allemagne) 2 %, Orange G 0,1 g/l ;
- réhydratation en tube à essai sous huile de paraffine pendant 6 à 72 heures à température ambiante d'une bande sèche d'Immobiline ayant un gradient de pH immobilisé non linéaire entre pH=3 et pH=10 (Pharmacia, Uppsalla, Suède) à l'aide des 500 µl d'échantillon dilué ;
- migration et focalisation des protéines de l'échantillon en fonction de leur point isoélectrique dans la bande d'Immobiline à l'aide du système Multiphore II (Pharmacia) conformément aux recommandations du fournisseur à 15 °C et pendant 100 kV/h à plus ou moins 10 % à 6000 V afin de réaliser une séparation selon la première dimension ;
- congélation de la bande d'Immobiline focalisée à -30 °C (facultatif) ;
- équilibration de la bande d'Immobiline pendant une durée de 15 minutes dans une solution Tris(hydroxyméthyl)-aminométhane 1,5 M, acide chlorhydrique pH=8,8 additionné d'urée 6 M, SDS 2 %, Glycérol 30 %, Bleu de Bromophénol 50 mg/ml et DTT 10 mg/ml ;
- équilibration de la bande d'Immobiline pendant 15 minutes en solution Tris(hydroxyméthyl)-aminométhane 1,5 M, acide chlorhydrique pH=8,8 additionné d'urée 6 M, SDS 2 %, Glycérol 30 %, Bleu de Bromophénol 50 mg/ml et Iodoacétamide 480 mg/ml ;
- égouttage pendant 5 à 10 minutes sur un papier buvard humide de l'excès de solution d'équilibration en déposant sur la tranche la bande d'immobiline ;
- dépôt de la bande d'Immobiline au sommet d'un gel de polyacrylamide C=2,8 %, T=12 % de 16 x 20 cm ;
- recouvrement de la bande d'Immobiline par une solution fondue d'agarose à 0.5 % en tampon Tris(hydroxyméthyl)-aminométhane 25 mM, glycine 192 mM, SDS 1 %, à pH=8,4 ;
- migration en fonction de leur poids moléculaire des protéines précédemment focalisées dans la bande d'Immobiline au sein du gel de polyacrylamide à l'aide du système Protean II xi (Bio-Rad, Hercules, Etats Unis d'Amérique) conformément aux recommandations du fournisseur afin de réaliser une séparation selon la deuxième dimension ;
- transfert des protéines du gel bi-dimensionnel vers une membrane Immobilon-P (Millipore, Bedford, Etats Unis d'Amérique) à l'aide du système Trans-blot (Biorad) dans une solution d'acide sulphonique 3-[cyclohexylamino]-1-propane (CAPS) 10 mM, méthanol 10 % à pH=11 pendant 16 à 24 heures à 400 mA ;
- passivation pendant 1 heure des sites de fixation restés libres sur la membrane à l'aide de la solution TNTL (solution de Tris(hydroxyméthyl)-aminométhane 15 mM, NaCl 0,14 M, Tween 20 à 0,5 ml/l, pH=8,0 additionnée de 5% de lait écrémé lyophilisé) ;
- incubation pendant 1 heure de la membrane dans une dilution à 10 µg/ml d'anticorps monoclonal de souris clone 13C9E9D6G8 (Bio-Mérieux) en solution TNTL ;
- lavage de la membrane 3 fois 10 minutes en solution TNTL ;
- incubation pendant 1 heure de la membrane dans une dilution au 1/5000 d'anticorps polyclonal de chèvre anti Fc de souris couplé à la peroxydase du raifort (Jackson ImmunoResearch, West Grove, Etats Unis d'Amérique) en solution TNTL ;
- lavage de la membrane 2 fois 10 minutes en solution TNTL ;
- lavage de la membrane 10 minutes en solution Tris(hydroxyméthyl)-aminométhane 0.1 M, acide chlorhydrique pH=7.6 ;
- révélation à l'aide du substrat chimiluminescent de la peroxydase du raifort Super Signal Ultra (Pierce, Rockford, Etats Unis d'Amérique) selon les recommandations du fournisseur ;
- détection de la lumière émise à l'aide du système FluorS Multi-Imager (Bio-Rad) pendant 1 minute à 1 heure avec une échelle de 4096 niveaux de gris ;
- quantification des formes de PSA libre clivé et non clivé à l'aide du logiciel Multi-Analyst (Bio-Rad) ;
- calcul des proportions de PSA libre clivé par rapport au PSA libre total, ou du PSA libre non clivé par rapport au PSA libre total, ou du PSA libre clivé par rapport au PSA libre non clivé, ou du PSA clivé par rapport au PSA total, ou du PSA clivé par rapport au PSA complexé.
- détermination du diagnostic du cancer de la prostate selon les valeurs de référence précédemment décrites.

La présente invention concerne donc une méthode de diagnostic d'un adénocarcinome de la prostate mais également une méthode permettant de différencier un patient atteint d'un adénocarcinome de la prostate, d'un patient atteint d'une pathologie bénigne de la prostate, comme par exemple une hypertrophie bénigne de la prostate (BPH).

Ainsi, comme cela est bien représenté à la figure 1, des ronds noirs et des ronds blancs sont représentés. Les ronds noirs représentent des cas de cancers alors que les ronds blancs concernent des BPH. On remarque essentiellement qu'il y a un mélange total entre les cas de cancers et les cas de BPH. Il est donc difficile avec un rapport de PSA libre sur PSA total de calculer réellement s'il y a cancer ou BPH. On remarque néanmoins un point important, à savoir que la très grande majorité des cas de cancers et de BPH, présentés par la suite, est située au-dessus d'une concentration de 2,1 ng/ml de PSA total. C'est le cas de soixante-quatorze des soixante-quinze sérums de patients testés. Seul le patient 35 comporte une valeur inférieure de 1,27 ng/ml. En règle générale, il sera donc logique de penser qu'en dessous de ce seuil, les cas de cancers ou de BPH sont très peu fréquents. Les patients présentant une concentration de PSA total inférieur à 2 ng/ml sont considérés comme étant sains. Le risque qu'ils aient une BPH n'est pas quantifiable car le nombre de cas analysés est trop faible pour être significatif.

La présente invention consiste donc à différencier cancer et BPH. Pour se faire, on effectue des électrophorèses bi-dimensionnelles comme cela est représenté par exemple sur la figure 2. On remarque trois protéines ou polypeptides de tailles différentes sur cette électrophorèse. Il y a tout d'abord en partie supérieure une protéine de sensiblement 34 kD, à plus ou moins 20 %, qui correspond en fait à la protéine de PSA non clivée.

En dessous deux masses sont représentées, à savoir sensiblement 26 kD et 21 kD, à plus ou moins 20 %, qui correspondent chacune à une forme clivée du PSA. Il existe une certaine proportion, entre les formes clivées et non clivées du PSA libre, qui peut être utilisée pour différencier cancer et BPH.

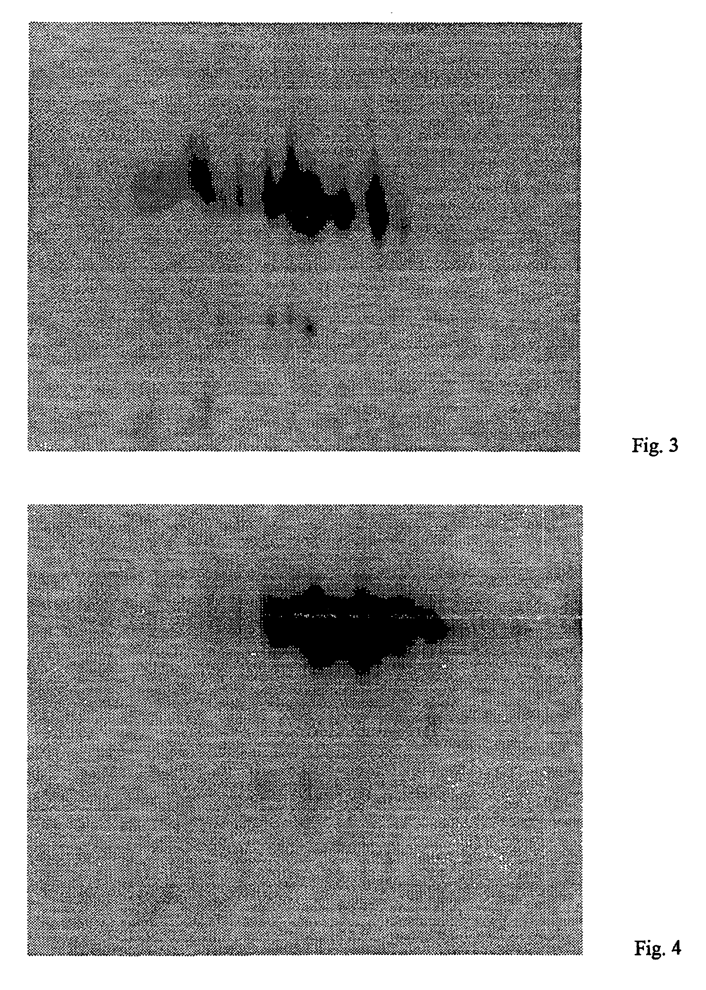

Ainsi, dans le cas de la figure 3, le patient est atteint d'une BPH et son sérum contient bien entendu plus de 2,1 ng/ml de PSA total, soit très exactement 3,9 ng/ml. Lorsqu'on rapporte cette concentration de PSA total sur la figure 1, on remarque qu'il y a, à ce niveau, à la fois des cas de BPH et des cas de cancers. Il s'avère que lorsque l'on fait la mesure du PSA libre total, c'est-à-dire clivé ou non clivé, que l'on mesure également le PSA libre clivé, on peut faire un ratio PSA libre clivé sur PSA libre total qui permet de diagnostiquer que le patient est atteint soit d'un cancer lorsque ce rapport est inférieur ou supérieur à une valeur de référence, soit d'une pathologie bénigne de la prostate lorsque ledit est rapport est supérieur ou inférieur à cette valeur.

De telles valeurs ont déjà été décrites ci-dessus et une telle méthode de diagnostic peut être appliquée également :

- à un rapport entre la concentration de PSA libre non clivé par rapport à la concentration du PSA libre total, ou
- à un ratio entre la concentration de PSA libre clivé par rapport à la concentration d u PSA libre non clivé, ou
- à un ratio entre la concentration de PSA libre clivé par rapport à la concentration du PSA total, ou enfin
- à un ratio entre la concentration de PSA libre clivé par rapport à la concentration du PSA complexé.

Il est également possible d'utiliser un ratio de ces rapports, c'est par exemple le cas en figure 6, avec le ratio entre le rapport de la concentration de PSA libre sur la concentration de PSA total en fonction du rapport de la concentration de PSA libre clivé sur la concentration de PSA libre non clivé. Ceci sera développé plus loin.

En règle générale, l'une de ces méthodes est à mettre en oeuvre lorsque la concentration, dans le sang ou dans le sérum, en PSA total est supérieure ou égale à 2 ng/ml.

Le procédé consiste donc à faire migrer les protéines issues du sang, du sérum, de l'urine ou du liquide séminal du patient, à détecter la présence et à quantifier le PSA libre sous forme clivée ou sous forme non clivée et à déterminer si le patient est sain ou atteint d'un adénocarcinome de la prostate ou d'une autre pathologie bénigne de la prostate en fonction des résultats obtenus précédemment.

La migration s'effectue généralement par électrophorèse bi-dimensionnelle et la détection par chimiluminescence avec révélation par Western Blot. Néanmoins, il est tout à fait possible d'envisager une technique utilisant des anticorps, qui sont soit spécifiques du PSA libre sous forme clivée soit spécifiques du PSA libre sous forme non clivée soit éventuellement du PSA lié, et qui ne nécessite pas une migration préalablement à la détection desdites formes.

L'ensemble des résultats obtenus est résumé sur le tableau ci-dessous :

| Sérum | Pathologie | TPSA | fPSA | cPSA | fPSA/TPSA | cPSA/ncPSA |
|---|---|---|---|---|---|---|
| Patient 1 | BPH | 3,90 | 0,87 | 0,20 | 22,31 | 30,55 |
| Patient 2 | BPH | 7,50 | 0,83 | 0,09 | 11,07 | 12,11 |
| Patient 3 | PCa | 11,00 | 1,21 | 0,00 | 11,00 | 0,00 |
| Patient 4 | PCa | 713,00 | 104,00 | 2,50 | 14,59 | 2,46 |
| Patient 5 | PCa | 3120,00 | 151,00 | 6,22 | 4,84 | 4,30 |
| Patient 6 | PCa | 4000,00 | 567,00 | 21,55 | 14,18 | 3,95 |
| Patient 7 | BPH | 9,13 | 2,84 | 0,04 | 31,11 | 1,37 |
| Patient 8 | BPH | 6,22 | 1,15 | 0,13 | 18,49 | 12,60 |
| Patient 9 | PCa | 7,07 | 1,24 | 0,03 | 17,54 | 2,61 |

(suite)

| Sérum | Pathologie | TPSA | fPSA | cPSA | fPSA/TPSA | cPSA/ncPSA |
|---|---|---|---|---|---|---|
| Patient 10 | BPH | 4,18 | 2,28 | 0,59 | 31,95 | 35,00 |
| Patient 11 | PCa | 10,5 | 0,84 | 0,00 | 8,00 | 0,00 |
| Patient 12 | BPH | 5,70 | 1,10 | 0,13 | 19,30 | 13,80 |
| Patient 13 | BPH | 9,68 | 2,82 | 0,42 | 29,13 | 17,66 |
| Patient 14 | PCa | 8,09 | 1,00 | 0,12 | 13,02 | 13,74 |
| Patient 15 | PCa | 10,55 | 2,09 | 0,00 | 19,81 | 0,00 |
| Patient 16 | PCa | 5,42 | 0,84 | 0,01 | 15,50 | 0,95 |
| Patient 17 | PCa | 10,00 | 0,69 | 0,04 | 6,90 | 6,30 |
| Patient 18 | PCa | 5,42 | 0,30 | 0,01 | 5,54 | 3,79 |
| Patient 19 | BPH | 5,55 | 1,00 | 0,04 | 18,02 | 4,64 |
| Patient 20 | BPH | 7,58 | 0,72 | 0,08 | 9,50 | 12,01 |
| Patient 21 | BPH | 4,16 | 0,97 | 0,06 | 23,32 | 6,56 |
| Patient 22 | BPH | 4,23 | 0,44 | 0,03 | 10,40 | 7,13 |
| Patient 23 | BPH | 8,27 | 3,50 | 0,42 | 42,32 | 13,80 |
| Patient 24 | PCa | 6,81 | 0,92 | 0,18 | 13,51 | 24,80 |
| Patient 25 | PCa | 29,83 | 1,73 | 0,00 | 5,80 | 0,00 |
| Patient 26 | PCa | 29,98 | 7,15 | 0,06 | 24,43 | 0,86 |
| Patient 27 | PCa | 4,98 | 0,29 | 0,00 | 5,82 | 0,00 |
| Patient 28 | BPH | 6,81 | 1,02 | 0,19 | 14,98 | 23,34 |
| Patient 29 | PCa | 5,66 | 0,70 | 0,03 | 12,37 | 5,13 |
| Patient 30 | PCa | 6,77 | 0,61 | 0,00 | 10,00 | 0,00 |
| Patient 31 | PCa | 7,9 | 1,79 | 0,02 | 22,66 | 0,97 |
| Patient 32 | PCa | 4,09 | 0,70 | 0,00 | 17,11 | 0,00 |
| Patient 33 | PCa | 26,51 | 3,16 | 0,00 | 11,92 | 0,00 |
| Patient 34 | PCa | 5,4 | 0,38 | 0,02 | 7,00 | 4,70 |
| Patient 35 | BPH | 1,27 | 0,19 | 0,05 | 14,18 | 31,72 |
| Patient 36 | BPH | 7,7 | 1,19 | 0,18 | 15,45 | 17,36 |
| Patient 37 | BPH | 5,24 | 0,72 | 0,06 | 13,74 | 9,51 |
| Patient 38 | BPH | 6,89 | 1,62 | 0,33 | 23,51 | 25,69 |
| Patient 39 | PCa | 9,58 | 1,23 | 0,00 | 12,84 | 0,36 |
| Patient 40 | BPH | 5,12 | 0,54 | 0,04 | 10,55 | 8,62 |
| Patient 41 | PCa | 7,07 | 1,24 | 0,02 | 17,54 | 1,84 |
| Patient 42 | BPH | 6,32 | 1,72 | 0,24 | 27,22 | 16,06 |
| Patient 43 | BPH | 7,67 | 2,57 | 0,28 | 33,51 | 12,42 |
| Patient 44 | PCa | 12,47 | 0,81 | 0,02 | 6,50 | 2,48 |
| Patient 45 | PCa | 10,57 | 0,70 | 0,00 | 6,62 | 0,00 |
| Patient 46 | PCa | 17,49 | 1,09 | 0,01 | 6,23 | 1,26 |
| Patient 47 | PCa | 10,84 | 2,56 | 0,00 | 18,29 | 0,00 |

(suite)

| Sérum | Pathologie | TPSA | fPSA | cPSA | fPSA/TPSA | cPSA/ncPSA |
|---|---|---|---|---|---|---|
| Patient 48 | PCa | 7,39 | 1,18 | 0,02 | 14,78 | 2,02 |
| Patient 49 | PCa | 40,39 | 10,00 | 0,00 | 24,76 | 0,00 |
| Patient 50 | PCa | 11,43 | 0,89 | 0,00 | 7,79 | 0,32 |
| Patient 51 | PCa | 46,46 | 3,43 | 0,14 | 7,38 | 4,17 |
| Patient 52 | BPH | 5,03 | 0,46 | 0,05 | 9,15 | 11,65 |
| Patient 53 | BPH | 4,01 | 0,52 | 0,10 | 12,97 | 25,11 |
| Patient 54 | PCa | 5,62 | 0,20 | 0,00 | 3,56 | 1,11 |
| Patient 55 | PCa | 9,5 | 0,34 | 0,00 | 3,58 | 1,07 |
| Patient 56 | BPH | 6,22 | 1,86 | 0,12 | 29,90 | 6,89 |
| Patient 57 | BPH | 2,42 | 0,74 | 0,07 | 22,73 | 9,71 |
| Patient 58 | BPH | 5,8 | 1,86 | 0,43 | 32,07 | 29,79 |
| Patient 59 | BPH | 4,52 | 1,05 | 0,09 | 23,23 | 9,01 |
| Patient 60 | BPH | 6,22 | 1,86 | 0,21 | 29,90 | 13,02 |
| Patient 61 | PCa | 4,63 | 0,71 | 0,00 | 15,33 | 0,00 |
| Patient 62 | PCa | 31,01 | 2,58 | 0,00 | 8,32 | 0,00 |
| Patient 63 | PCa | 33,96 | 10,00 | 0,00 | 29,45 | 0,00 |
| Patient 64 | PCa | 58,82 | 6,84 | 0,04 | 11,63 | 0,56 |
| Patient 65 | PCa | 20,29 | 3,15 | 0,09 | 11,04 | 2,92 |
| Patient 66 | BPH | 5,01 | 1,17 | 0,08 | 23,35 | 7,10 |
| Patient 67 | BPH | 16 | 2,24 | 0,70 | 14,00 | 45,13 |
| Patient 68 | BPH | 4,55 | 1,01 | 0,07 | 22,20 | 8,01 |
| Patient 69 | BPH | 5,20 | 1,53 | 0,13 | 29,42 | 9,34 |
| Patient 70 | BPH | 7,21 | 1,43 | 0,17 | 19,83 | 13,77 |
| Patient 71 | PCa | 5,56 | 0,86 | 0,00 | 15,47 | 0,00 |
| Patient 72 | PCa | 5,62 | 0,20 | 0,01 | 3,56 | 3,45 |
| Patient 73 | PCa | 98,82 | 7,68 | 0,03 | 7,77 | 0,33 |
| Patient 74 | PCa | 18,41 | 1,64 | 0,08 | 8,78 | 4,87 |
| Patient 75 | BPH | 4,29 | 1,48 | 0,22 | 34,50 | 17,71 |

Il s'agit d'une étude comparative entre les sérums de soixante quinze patients différents.

Afin de bien comprendre le tableau ci-dessus, il convient de noter que les concentrations de PSA total (TPSA), PSA libre (fPSA) et PSA clivé (cPSA) sont exprimées en ng/ml. Les ratios, pour leur part, sont exprimés en pourcentage.

Ce tableau permet de visualiser rapidement les ratios qui peuvent exister entre :

- la concentration de PSA total, ci-après référencé TPSA,
- la concentration de PSA lié à des inhibiteurs de protéases, telles que l'ACT, ci-après référencé bPSA,
- la concentration de PSA libre, ci-après référencé fPSA,
- la concentration de PSA libre non clivé, ci-après référencé ncPSA, et
- la concentration de PSA libre clivé, ci-après référencé cPSA.

Il convient de noter que l'on utilise dans la colonne de droite la concentration de PSA non clivé (ncPSA). Même si celle-ci n'est pas donnée, elle se déduit aisément de l'équation suivante :

EP 1 095 276 B1

$$ncPSA = fPSA - cPSA$$

Lorsque l'on prend en compte le ratio fPSA/TPSA, on remarque que ce ratio n'est pas du tout caractéristique. Ainsi, sur les six premiers patiente, seuls les patients 1 et 5 ont un ratio caractéristique, c'est-à-dire qu'il pourrait permettre de distinguer un cas de cancer d'une cas de BPH.

Dans les autres cas, les ratios sont compris entre 11,00 et 14,59. Une discrimination n'est donc pas possible entre cancer et BPH. Ceci est également vrai avec les autres sérums de patients. Il est par exemple très difficile de différencier les patients 8 et 9, 12 et 15, etc.

Des exemples particulièrement frappants sont les cas des patients 22, 40 et 52 qui sont atteints d'une BPH avec un ratio fPSA/TPSA toujours inférieur à 11, alors que dans le même temps, les patients 26, 31, 49 et 63 sont atteints d'un cancer en ayant un ratio toujours supérieur à 22. Entre ces valeurs 11 et 22 il y a donc une zone floue quant au diagnostic. Or de nombreux sérums sont dans cette zone (voire au-delà comme c'est le cas des sept patients que l'on vient de décrire). Le nombre de sérums compris dans cette zone est de trente et un, soit un total de trente-huit sérums non diagnostiqués avec une probabilité efficiente. Ce qui représente une erreur de sensiblement 50,67 %. L'utilisation du rapport fPSA/TPSA est dans ce cas inadaptée à la distinction entre BPH et cancer.

Ceci montre bien les limites de cette technique.

Si par contre, on utilise les rapport existants entre cPSA/bPSA, cPSA/TPSA, ncPSA/fPSA, cPSA/fPSA et cPSA/ncPSA, et en se reportant toujours aux six premiers sérums de patients, on remarque qu'il existe une différence importante entre les ratios des patients 1 et 2 atteints d'une BPH et les patients 3 à 6 atteints d'un cancer.

Ces rapports qui utilisent les formes clivées et/ou non clivées du PSA sont donc bien plus représentatifs de l'état réel d'un patient.

Ainsi, dans le cadre des exemples tirés du tableau, avec le rapport cPSA/TPSA, dans le cas d'une BPH le rapport est toujours supérieur ou égal à 1,2 % et dans le cas d'un cancer le rapport est toujours inférieur à 0,54 %.

Avec le rapport cPSA/bPSA, dans le cas d'une BPH le rapport est toujours supérieur à 1,34 % et dans le cas d'un cancer le rapport est toujours inférieur à 0,63 %.

Avec le rapport ncPSA/fPSA, dans le cas d'une BPH le rapport est toujours inférieur à 89,2 % et dans le cas d'un cancer le rapport est toujours supérieur à 95,88 %.

Avec le rapport cPSA/fPSA, dans le cas d'une BPH le rapport est toujours supérieur à 10,8 % et dans le cas d'un cancer le rapport est toujours inférieur à 4,12 %.

Enfin, avec le rapport cPSA/ncPSA, dans le cas d'une BPH le rapport est toujours supérieur à 12,11 % et dans le cas d'un cancer le rapport est toujours inférieur à 4,3 %.

Les rapports, qui ne sont pas représentatifs, sont constitués par les rapports ncPSA/TPSA, ncPSA/bPSA et fPSA/TPSA puisque les ratios de trois patients 3, 4 et 6, atteints de cancer, se situent dans l'intervalle délimité par les ratios des patients 1 et 2, atteints d'une BPH.

De ce fait, il peut être particulièrement intéressant d'utiliser une combinaison des différents rapports cPSA/TPSA, cPSA/bPSA, ncPSA/fPSA, cPSA/fPSA et cPSA/ncPSA, voire la totalité de ces rapports, pour différencier une pathologie bénigne de la prostate par rapport à un cancer de la prostate.

Lorsque l'on met en exergue les patients 22, 40 et 52, on remarque que leur rapport cPSA/ncPSA est respectivement de 7,13 - 8,62 - 11,65. En ce qui concerne les patients 26, 31, 49 et 63, leur rapport cPSA/ncPSA est alors respectivement de 0,86 - 0,97 - 0,00 - 0,00.

Le rapport est donc parfaitement adapté au diagnostic que les praticiens souhaitent réalisés.

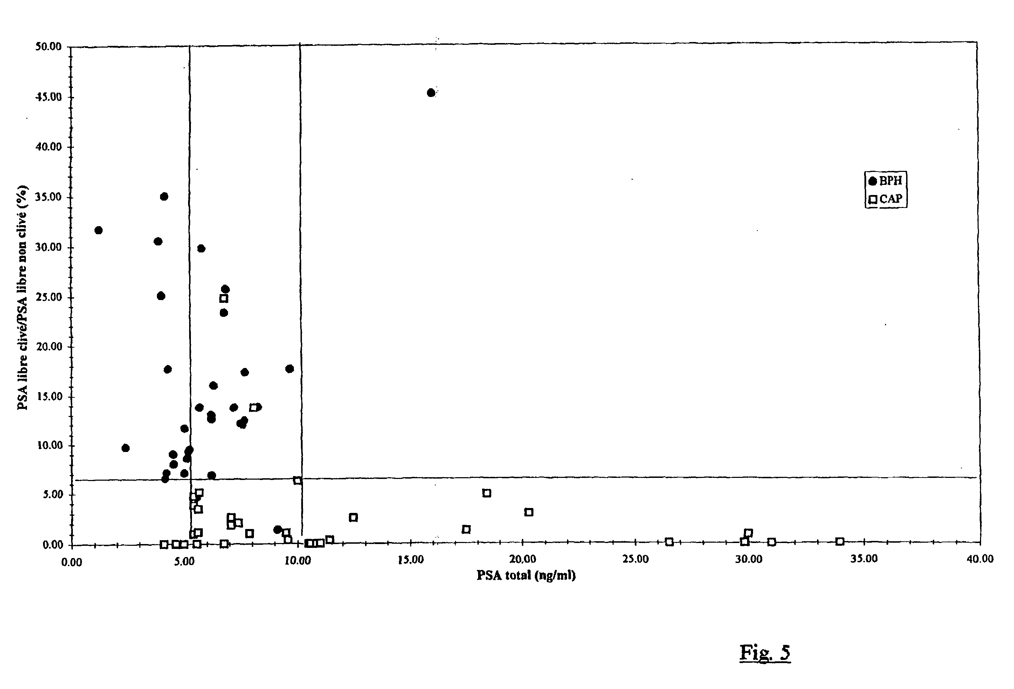

Si l'on se réfère maintenant à la figure 5, on remarque qu'au-dessus d'une droite horizontale correspondant à 6,5 % du ratio cPSA/ncPSA, la quasi totalité des cas de BPH est représentée, à l'exception de deux cas. Il s'agit des patients 7 et 19 dont les rapports sont de 1,37 et 4,64, soit deux cas sur trente-trois, c'est-à-dire que l'erreur est de sensiblement 6 %, ce qui est considérablement inférieur au rapport classique fPSA/TPSA, vu précédemment.

On remarque également qu'au-dessous de cette droite horizontale de référence située à 6,5 % du ratio cPSA/ncPSA, la quasi totalité des cas de cancer est représentée, à l'exception de deux cas. Il s'agit des patients 14 et 24 dont les rapports sont de 13,74 et 24,80, soit deux cas sur quarante-deux, c'est-à-dire que l'erreur est de sensiblement 4,77%.

La figure 6 est encore plus parlante. On remarque deux droites, l'une verticale l'autre horizontale, qui se croisent. La droite verticale correspond à la valeur 15,1 % du rapport fPSA/TPSA et la droite horizontale correspond à la valeur 6,2 % du rapport cPSA/ncPSA. Quatre cas de figure peuvent être déduit, il s'agit de :

- ce rapport cPSA/ncPSA a une valeur inférieure ou égale à 6,2 % et ce rapport fPSA/TPSA a une valeur inférieure ou égale à 15,1 %, de sorte que le patient est atteint d'un adénocarcinome de la prostate,
- ce rapport cPSA/ncPSA a une valeur supérieure à 6,2 % et ce rapport fPSA/TPSA a une valeur supérieure à 15,1 %, de sorte que le patient est atteint d'une pathologie bénigne de la prostate, telle qu'une hypertrophie bénigne de la prostate,

- ce rapport cPSA/ncPSA a une valeur inférieure ou égale à 6,2 % et ce rapport fPSA/TPSA a une valeur supérieure à 15,1 %, de sorte que le patient a une forte probabilité d'être atteint d'un adénocarcinome de la prostate et une faible probabilité d'être atteint d'une pathologie bénigne de la prostate, telle qu'une hypertrophie bénigne de la prostate,
- ce rapport cPSA/ncPSA a une valeur supérieure à 6,2 % ce rapport fPSA/TPSA a une valeur inférieure ou égale à 15,1 %, de sorte que le patient a une forte probabilité d'être atteint d'une pathologie bénigne de la prostate, telle qu'une hypertrophie bénigne de la prostate, et une faible probabilité d'être atteint d'un adénocarcinome de la prostate.

Même si les valeurs de référence sont données à 6,2 et 15,1 %, il est possible qu'avec un autre panel de sérums ou bien un nombre différents de sérums ces chiffres varient. Donc préférentiellement, la première valeur de référence est comprise entre 1 et 18 %, préférentiellement entre 5 et 8 %, et la seconde valeur de référence est comprise entre 10 et 20 %, préférentiellement entre 13 et 17 %.

En ce qui concerne les probabilités évoqués plus haut, dans le troisième cas décrit ci-dessus, la forte probabilité d'être atteint d'un adénocarcinome de la prostate est comprise entre 84 et 87 % et la faible probabilité d'être atteint d'une pathologie bénigne de la prostate est comprise entre 13 et 16 %. Ainsi, il y a deux cas de BPH sur quatorze au total. Néanmoins pour ces troisième et quatrième cas, ces valeurs peuvent être élargies du fait qu'avec d'autres échantillons une fourchette plus large peut être trouvée, par exemple 80 à 95 % pour la forte probabilité, et 5 à 20 % pour la faible probabilité.

Dans le quatrième cas, la forte probabilité d'être atteint d'une pathologie bénigne de la prostate est comprise entre 82 et 85 %, et la faible probabilité d'être atteint d'un adénocarcinome de la prostate est comprise entre 15 et 18 %. Ce qui correspond à deux cas de cancers pour douze cas au total.

Néanmoins pour ces troisième et quatrième cas, ces valeurs peuvent être élargies du fait qu'avec d'autres échantillons une fourchette plus large peut être trouvée, par exemple 80 à 95 % pour la forte probabilité, et 5 à 20 % pour la faible probabilité.

Nous disposons de trois variables indépendantes :

- A = bPSA, c'est-à-dire le PSA complexé ou lié à des inhibiteurs (ACT...)
- B = ncPSA, c'est-à-dire le PSA libre non clivé
- C = cPSA, c'est-à-dire le PSA libre clivé

avec des concentrations telles que A >> B >> C

En conséquence, les différents ratios peuvent être écrits de la façon qui suit et conduire aux approximations suivantes :

$$1. \quad \frac{fPSA}{TPSA} = \frac{B+C}{A+B+C} \approx \frac{B}{A}$$

$$2. \quad \frac{ncPSA}{TPSA} = \frac{B}{A+B+C} \approx \frac{B}{A}$$

$$3. \quad \frac{cPSA}{TPSA} = \frac{C}{A+B+C} \approx \frac{C}{A}$$

$$4. \quad \frac{ncPSA}{bPSA} = \frac{B}{A}$$

$$5. \quad \frac{cPSA}{bPSA} = \frac{C}{A}$$

$$6. \quad \frac{ncPSA}{fPSA} = \frac{B}{B+C} = 1 - \frac{C}{B+C} = 1 - \frac{1}{1+\frac{B}{C}} \approx 1 - \frac{C}{B}$$

$$7. \quad \frac{cPSA}{fPSA} = \frac{C}{B+C} = \frac{1}{1+\frac{B}{C}} \approx \frac{C}{B}$$

$$8. \quad \frac{cPSA}{ncPSA} = \frac{C}{B}$$

Il apparaît donc que les ratios 1, 2 et 4 conduisent au même type de réponse. Le ratio 1 a déjà été décrit dans l'état de la technique, qui a été discuté précédemment, et conduit à de nombreux diagnostics erronés, par conséquent, les ratios 2 et 4 ne sont pas intéressants.

Les ratios 6, 7 et 8 sont reliés par une relation mathématique exacte ce qui revient à dire qu'ils permettent d'aboutir à une réponse identique.

Les ratios 3 et 5 conduisent à une réponse équivalente différente des ratios précédents.

Les ratios 3 et 5 à 8 sont donc représentatifs.

Il existe plusieurs stratégies de dosage du PSA libre clivé ou non clivé.

Le PSA clivé présente les caractéristiques suivantes. Premièrement, les sites de fragmentations internes n'affectent pas la cohésion tridimensionnelle de la molécule du fait de l'existence de cinq ponts disulfures qui lient les différents fragments. Deuxièmement, après réduction, ces ponts disulfures sont rompus et les différents fragments de PSA se dissocient. Troisièmement, les fragmentations modifient la conformation du PSA et lui font perdre son activité enzymatique et sa capacité de liaison aux inhibiteurs de protéase.

I - Dosage des formes clivées après réduction de l'échantillon :

Après fragmentation ou clivage et réduction, des zones internes au PSA sont démasquées et des épitopes inaccessibles sur le PSA entier sont dévoilés. Du PSA clivé et réduit ou des peptides situés avant et après les sites de clivage comportent de tels épitopes. Des immunisations avec ces peptides ou du PSA clivé et réduit permettent d'obtenir des anticorps dirigés contre les épitopes recherchés. Après réduction de l'échantillon à analyser, un test utilisant de tels anticorps dose spécifiquement le PSA clivé.

Ainsi, un immunogène de PSA clivé et réduit a été obtenu et caractérisé. Des peptides situés avant ou après les sites de clivages ont été synthétisés. Une immunisation avec du PSA clivé et réduit a été réalisée sur souris. Dix clones d'anticorps monoclonaux spécifiques des épitopes propres au PSA clivé ont été obtenus

II - Dosage des formes clivées sans réduction de l'échantillon :

Le PSA clivé est inactif et peut comporter une modification conformationnelle responsable de la perte d'activité. Une immunisation sur souris avec ce PSA peut permettre d'obtenir des anticorps dirigés contre ces épitopes conformationnels. Un test utilisant ces anticorps dose directement et spécifiquement le PSA clivé.

Du PSA inactif majoritairement clivé a été obtenu chez un fournisseur. Une immunisation sur souris a été réalisée.

III - Dosage du PSA non clivé après réduction de l'échantillon :

Après réduction de l'échantillon, le PSA clivé se fragmente alors que l'enchaînement d'acide aminé du PSA non clivé reste intact. Des peptides C-terminal et N-terminal permettent d'effectuer une mise en évidence spécifiquement des anticorps dirigés contre la partie C-terminal ou la partie N-terminal ou encore d'en obtenir après immunisation. Après réduction de l'échantillon, un sandwich avec un anticorps dirigé contre la partie C-terminal et un anticorps dirigé contre la partie C-terminal (***???) permettrait de ne reconnaître que le PSA entier.

Des peptides C-terminal et N-terminal du PSA ont été réalisés. Ces peptides ont été utilisés pour mettre en évidence des immunisations sur souris réalisées avec du PSA séminal ou pour immuniser directement des souris. Des anticorps ont été mis en évidence pour définir des couples reconnaissant le PSA réduit non clivé et non le PSA réduit clivé.

Nous disposons de certains couples d'anticorps disposant des caractéristiques attendues comme cela est bien représenté sur le tableau joint ci-après :

| Anticorps monoclonal | PSA réduit non clivé à 100 ng/ml | PSA réduit clivé à 100 ng/ml |
|---|---|---|
| 4H10B4 | >2 | 0,133 |
| 21D7C1 | >2 | 0,103 |
| 1C7H10 | >2 | 0,121 |
| 12E6H9 | 1,803 | 0,082 |

IV - Dosage du PSA non clivé en condition non réduite :

Le PSA non clivé peut comporter certains épitopes conformationnels différents du PSA clivé (référence au chapitre II). Des peptides chevauchants les sites de fragmentation peuvent présenter de tels épitopes. Une immunisation sur souris avec ces peptides ou avec du PSA non clivé permet d'obtenir des anticorps dirigés contre ces épitopes. Un test, utilisant ces anticorps, dose directement et spécifiquement le PSA non clivé.
Les peptides ont été synthétisés. Une immunisation avec du PSA majoritairement non clivé a été réalisée sur souris.

V - Dosage du PSA enzymatiquement actif :

Le PSA non clivé dans le sérum peut être enzymatiquement actif. Ainsi, il a été démontré que le PSA clivé était enzymatiquement inactif mais pas l'inverse. Un peptide inhibiteur présentant une forte affinité pour le PSA actif peut être conçu par phage display et/ou modification de séquences naturelles. Le peptide ainsi conçu peut être utilisé pour capturer sélectivement le PSA enzymatiquement actif et permettre ainsi son dosage.
Le protocole qui suit permet le dosage spécifique du PSA non clivé. Il consiste à effectuer les opérations suivantes :

- Revêtement de micro-plaque ELISA par 100µl d'anticorps à 20 µg/ml en tampon Tris Maléate 0.2M, pH=6.2.
- Incubation 1 nuit à température ambiante.
- Lavage par 3 fois 300 µl de tampon Tris Maléate 0.2M, Tween 20 0.5%, pH=6.2
- Passivation 1 heure par 200 µl de tampon Tris Maléate 0.2M, sérum de veau foetal 3 %, pH=6.2.
- Lavage par 3 fois 300 µl de tampon Tris Maléate 0.2M, Tween 20 0.5%, pH=6.2
- Dilution du PSA à 100 ng/ml en tampon Tris Maléate 0.2M, sérum de veau foetal 3 %, pH=6.2.
- Incubation de 100 µl de PSA 1 heure à 37°C.
- Lavage par 3 fois 300 µl de tampon Tris Maléate 0.2M, Tween 20 0.5%, pH=6.2
- Incubation 1 heure à 37°C de 100 µl d'anticorps anti-PSA biotinylé 3A1B5, dilué 1/1000 en tampon Tris Maléate 0.2M, sérum de veau foetal 3 %, pH=6.2.
- Lavage par 3 fois 300 µl de tampon Tris Maléate 0.2M, Tween 20 0.5%, pH=6.2
- Incubation 1 heure à 37°C de 100 µl de streptavidine-phosphatase alcaline, dilué 1/500 en tampon Tris 0.2M, NaCl 0.1M, $MgCl_2$ 1mM, $ZnCl_2$ 0.1 mM, sérum de veau foetal 3 %, pH=6.5.
- Lavage par 3 fois 300 µl de tampon Tris Maléate 0.2M, Tween 20 0.5%, pH=6.2
- Révélation 15 minutes à 37°C avec 100 µl de substrat pNPP
- Blocage de la réaction par 100 µl de soude 1N.
- Lecture sur photospectromètre à 405 nm avec soustraction du bruit de fond à 630 nm.

**Revendications**

1. Méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, **caractérisée en ce qu'**elle consiste à :

   - mesurer le niveau de PSA libre total, c'est-à-dire clivé et non clivé,
   - mesurer le niveau de PSA libre clivé,
   - calculer la proportion de PSA libre clivé par rapport au PSA libre total, et
   - distinguer deux cas, à savoir :

      • ce rapport a une valeur inférieure ou égale à une valeur de référence ou de différenciation, trouvée chez des patients affectés par un adénocarcinome de la prostate, de sorte que le patient est atteint d'un adénocarcinome de la prostate,
      • ce rapport a une valeur supérieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

2. Méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, **caractérisée en ce qu'**elle consiste à :

- mesurer le niveau de PSA libre non clivé,
- mesurer le niveau de PSA libre clivé,
- calculer la proportion de PSA libre clivé par rapport au PSA libre non clivé, et
- distinguer deux cas, à savoir :

  • ce rapport a une valeur inférieure ou égale à une valeur de référence ou de différenciation, trouvée chez des patients affectés par un adénocarcinome de la prostate, de sorte que le patient est atteint d'un adénocarcinome de la prostate,
  • ce rapport a une valeur supérieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

**3.** Méthode, selon l'une quelconque des revendications 1 ou 2, **caractérisée en ce que** la valeur de référence est comprise entre 2 et 12 % et préférentiellement entre 5 et 8 %.

**4.** Méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, **caractérisée en ce qu'**elle consiste à :

- mesurer le niveau de PSA libre total, c'est-à-dire clivé et non clivé,
- mesurer le niveau de PSA libre non clivé,
- calculer la proportion de PSA libre non clivé par rapport au PSA libre total, et
- distinguer deux cas, à savoir :

  • ce rapport a une valeur supérieure ou égale à une valeur de référence ou de différenciation, trouvée chez des patients affectés par un adénocarcinome de la prostate, de sorte que le patient est atteint d'un adénocarcinome de la prostate,
  • ce rapport a une valeur inférieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

**5.** Méthode, selon la revendication 4, **caractérisée en ce que** la valeur de référence est comprise entre 88 et 98 % et préférentiellement entre 93 et 95 %.

**6.** Méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, **caractérisée en ce qu'**elle consiste à :

- mesurer le niveau de PSA total, c'est-à-dire complexé et libre, clivé et non clivé,
- mesurer le niveau de PSA libre clivé,
- calculer la proportion de PSA libre clivé par rapport au PSA total, et
- distinguer deux cas, à savoir :

  • ce rapport a une valeur inférieure ou égale à une valeur de référence ou de différenciation, trouvée chez des patients affectés par un adénocarcinome de la prostate, de sorte que le patient est atteint d'un adénocarcinome de la prostate,
  • ce rapport a une valeur supérieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

**7.** Méthode, selon la revendication 6, **caractérisée en ce que** la valeur de référence est comprise entre 0,1 et 2 % et préférentiellement entre 0,4 et 1 %.

**8.** Méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, **caractérisée en ce qu'**elle consiste à :

- mesurer le niveau de PSA complexé,

- mesurer le niveau de PSA libre clivé,
- calculer la proportion de PSA libre clivé par rapport au PSA complexé, et
- distinguer deux cas, à savoir :

  • ce rapport a une valeur supérieure ou égale à une valeur de référence ou de différenciation, trouvée chez des patients affectés par un adénocarcinome de la prostate, de sorte que le patient est atteint d'un adénocarcinome de la prostate,
  • ce rapport a une valeur inférieure à la valeur de référence, trouvée chez des patients affectés par une pathologie bénigne de la prostate, de sorte que le patient est atteint d'une pathologie bénigne de la prostate, telle qu'une hypertrophie bénigne de la prostate.

**9.** Méthode, selon la revendication 8, **caractérisée en ce que** la valeur de référence est comprise entre 0,1 et 2,2 % et préférentiellement entre 0,6 et 1,2 %.

**10.** Méthode d'analyse en vue du diagnostic d'un adénocarcinome de la prostate chez un patient humain mâle, sans pratiquer de biopsie prostatique, qui utilise la protéine PSA (prostate specific antigen) présente dans un échantillon de sang, de sérum, d'urine ou de liquide séminal du patient, **caractérisée en ce qu'**elle consiste à:

- mesurer le niveau de PSA libre non clivé,
- mesurer le niveau de PSA libre clivé,
- mesurer le niveau de PSA libre,
- mesurer le niveau de PSA total,
- calculer la proportion de PSA libre clivé par rapport au PSA libre non clivé, dit premier rapport,
- calculer la proportion de PSA libre par rapport au PSA total, dit second rapport, et
- distinguer quatre cas, à savoir:

  • ce premier rapport a une valeur inférieure ou égale à une première valeur de référence ou de différenciation et ce second rapport a une valeur inférieure ou égale à une seconde valeur de référence ou de différenciation, de sorte que le patient est atteint d'un adénocarcinome de la prostate,
  • ce premier rapport a une valeur supérieure ou égale à une première valeur de référence ou de différenciation et ce second rapport a une valeur supérieure ou égale à une seconde valeur de référence ou de différenciation, de sorte que le patient est atteint d'une pathologie bénigne de la prostate, telle qu'une hypertrophie bénigne de la prostate,
  • ce premier rapport a une valeur inférieure ou égale à une première valeur de référence ou de différenciation et ce second rapport a une valeur supérieure ou égale à une seconde valeur de référence ou de différenciation, de sorte que le patient a une forte probabilité d'être atteint d'un adénocarcinome de la prostate et une faible probabilité d'être atteint d'une pathologie bénigne de la prostate, telle qu'une hypertrophie bénigne de la prostate,
  • ce premier rapport a une valeur supérieure ou égale à une première valeur de référence ou de différenciation et ce second rapport a une valeur inférieure ou égale à une seconde valeur de référence ou de différenciation, de sorte que le patient a une forte probabilité d'être atteint d'une pathologie bénigne de la prostate, telle qu'une hypertrophie bénigne de la prostate, et une faible probabilité d'être atteint d'un adénocarcinome de la prostate.

**11.** Méthode, selon la revendication 10, **caractérisée en ce que** la première valeur de référence est comprise entre 1 et 18 %, préférentiellement entre 5 et 8 % et plus précisément de sensiblement 6,3 %, et que la seconde valeur de référence est comprise entre 10 et 20 %, préférentiellement entre 13 et 17 % et plus précisément de sensiblement 15 %.

**12.** Méthode, selon l'une quelconque des revendications 10 ou 11, **caractérisée en ce que** la forte probabilité d'être atteint d'un adénocarcinome de la prostate est comprise entre 80 et 95 % et préférentiellement entre 84 et 87 % et la faible probabilité d'être atteint d'une pathologie bénigne de la prostate est comprise entre 5 et 20 % et préférentiellement entre 13 et 16 %, et que la forte probabilité d'être atteint d'une pathologie bénigne de la prostate est comprise entre 80 et 95 % et préférentiellement entre 82 et 85 %, et la faible probabilité d'être atteint d'un adénocarcinome de la prostate est comprise entre 5 et 20 % et préférentiellement entre 15 et 18 %.

**13.** Méthode d'analyse en vue du diagnostic d'un adénocarcinome ou d'une pathologie bénigne de la prostate chez un patient, qui utilise la combinaison d'au moins deux méthodes, chaque méthode étant constituée par l'une quelconque des revendications 1 ou 2 ou 3, ou bien 4 ou 5, ou bien 6 ou 7, ou bien 8 ou 9, ou bien 10 ou 11 ou 12.

14. Méthode, selon l'une quelconque des revendications 1 à 13, **caractérisée en ce qu'**elle est mise en oeuvre lorsque la concentration en PSA total dans le sang ou dans le sérum est supérieure à 2 ng/ml.

15. Procédé utilisant une méthode, selon l'une quelconque des revendications 1 à 9, **caractérisé en ce qu'**il consiste à :

- détecter la présence et quantifier le niveau de deux des paramètres suivants : PSA libre sous forme clivée, PSA libre sous forme non clivée, PSA complexé et PSA total,
- déterminer la valeur de la proportion de :

  • PSA libre clivé par rapport au PSA libre total,
  • PSA libre clivé par rapport au PSA libre non clivé,
  • PSA libre non clivé par rapport au PSA libre total,
  • PSA libre clivé par rapport au PSA total, ou
  • PSA libre clivé par rapport au PSA complexé,

  et

- comparer cette proportion par rapport à une valeur de référence, en vue du diagnostic d'un adénocarcinome de la prostate chez un patient humain mâle.

16. Procédé, selon la revendication 15, **caractérisé en ce que** préalablement on fait migrer les protéines issues du sang, du sérum, l'urine ou du liquide séminal du patient.

17. Procédé, selon l'une quelconque des revendications 15 ou 16, **caractérisé en ce que** la migration s'effectue par électrophorèse bi-dimensionnelle.

18. Procédé, selon l'une quelconque des revendications 15 à 17, **caractérisé en ce que** préalablement on dose le taux de PSA total.

19. Procédé, selon l'une quelconque des revendications 15 ou 18, **caractérisé en ce que** la détection s'effectue par chimiluminescence après révélation par Western Blot.


**Patentansprüche**

1. Analyseverfahren für die Diagnose eines Adenokarzinoms der Prostata bei einem männlichen menschlichen Patienten, ohne Vornahme einer Prostatabiospie, bei dem das in einer Blut-, Serum-, Urin- oder Samenflüssigkeitsprobe des Patienten vorhandene PSA(prostataspezifische Antigen)-Protein verwendet wird, **dadurch gekennzeichnet, daß** es besteht aus:

- dem Messen des Niveaus von gesamtem freien PSA, also gespaltenem und nicht-gespaltenem PSA,

- dem Messen des Niveaus von gespaltenem freien PSA,

- dem Berechnen des Verhältnisses von gespaltenem freien PSA zu gesamtem freien PSA, und

- dem Unterscheiden von zwei Fällen, nämlich:

  • dieses Verhältnis hat einen Wert, der kleiner oder gleich einem Referenz- oder Unterscheidungswert ist, der sich bei Patienten mit einem Adenokarzinom der Prostata ergibt, so daß der Patient von einem Adenokarzinom der Prostata betroffen ist,

  • dieses Verhältnis hat einen Wert, der größer als der Referenzwert ist, der sich bei Patienten mit gutartiger Pathologie der Prostata ergibt, so daß der Patient von einer gutartigen Pathologie der Prostata, wie z.B. einer gutartigen Prostatahypertrophie, betroffen ist.

**2.** Analyseverfahren für die Diagnose eines Adenokarzinoms der Prostata bei einem männlichen menschlichen Patienten, ohne Vornahme einer Prostatabiospie, bei dem das in einer Blut-, Serum-, Urin- oder Samenflüssigkeitsprobe des Patienten vorhandene PSA(prostataspezifische Antigen)-Protein verwendet wird, **dadurch gekennzeichnet, daß** es besteht aus:

- dem Messen des Niveaus von nicht-gespaltenem freien PSA,

- dem Messen des Niveaus von gespaltenem freien PSA,

- dem Berechnen des Verhältnisses von gespaltenem freien PSA zu nicht-gespaltenem freien PSA, und

- dem Unterscheiden von zwei Fällen, nämlich:

  • dieses Verhältnis hat einen Wert, der kleiner oder gleich einem Referenz- oder Unterscheidungswert ist, der sich bei Patienten mit einem Adenokarzinom der Prostata ergibt, so daß der Patient von einem Adenokarzinom der Prostata betroffen ist,

  • dieses Verhältnis hat einen Wert, der größer als der Referenzwert ist, der sich bei Patienten mit gutartiger Pathologie der Prostata ergibt, so daß der Patient von einer gutartigen Pathologie der Prostata, wie z.B. einer gutartigen Prostatahypertrophie, betroffen ist.

**3.** Verfahren nach Anspruch 1 oder 2, **dadurch gekennzeichnet, daß** der Referenzwert bei 2 bis 12 % und vorzugsweise bei 5 bis 8 % liegt.

**4.** Analyseverfahren für die Diagnose eines Adenokarzinoms der Prostata bei einem männlichen menschlichen Patienten, ohne Vornahme einer Prostatabiospie, bei dem das in einer Blut-, Serum-, Urin- oder Samenflüssigkeitsprobe des Patienten vorhandene PSA(prostataspezifische Antigen)-Protein verwendet wird, **dadurch gekennzeichnet, daß** es besteht aus:

- dem Messen des Niveaus von gesamtem freien PSA, also gespaltenem und nicht-gespaltenem PSA,

- dem Messen des Niveaus von nicht-gespaltenem freien PSA,

- dem Berechnen des Verhältnisses von nicht-gespaltenem freien PSA zu gesamtem freien PSA, und

- dem Unterscheiden von zwei Fällen, nämlich:

  • dieses Verhältnis hat einen Wert, der größer oder gleich einem Referenz- oder Unterscheidungswert ist, der sich bei Patienten mit einem Adenokarzinom der Prostata ergibt, so daß der Patient von einem Adenokarzinom der Prostata betroffen ist,

  • dieses Verhältnis hat einen Wert, der kleiner als der Referenzwert ist, der sich bei Patienten mit einer gutartigen Pathologie der Prostata ergibt, so daß der Patient von einer gutartigen Pathologie der Prostata, wie z.B. einer gutartigen Prostatahyperthropie, betroffen ist.

**5.** Verfahren nach Anspruch 4, **dadurch gekennzeichnet, daß** der Referenzwert bei 88 bis 98 % und vorzugsweise bei 93 bis 95 % liegt.

**6.** Analyseverfahren für die Diagnose eines Adenokarzinoms der Prostata bei einem männlichen menschlichen Patienten, ohne Vornahme einer Prostatabiospie, bei dem das in einer Blut-, Serum-, Urin- oder Samenflüssigkeitsprobe des Patienten vorhandene PSA(prostataspezifische Antigen)-Protein verwendet wird, **dadurch gekennzeichnet, daß** es besteht aus:

- dem Messen des Niveaus von gesamtem PSA, also komplexiertem und freiem, gespaltenem und nicht-gespaltenem PSA,

- dem Messen des Niveaus von gespaltenem freien PSA,

- dem Berechnen des Verhältnisses von gespaltenem freien PSA zu gesamtem PSA, und

- dem Unterscheiden von zwei Fällen, nämlich:

  • dieses Verhältnis hat einen Wert, der kleiner oder gleich einem Referenz- oder Unterscheidungswert ist, der sich bei Patienten mit einem Adenokarzinom der Prostata ergibt, so daß der Patient von einem Adenokarzinom der Prostata betroffen ist,

  • dieses Verhältnis hat einen Wert, der größer als der Referenzwert ist, der sich bei Patienten mit gutartiger Pathologie der Prostata ergibt, so daß der Patient von einer gutartigen Pathologie der Prostata, wie z.B. einer gutartigen Prostatahyperthropie, betroffen ist.

7. Verfahren nach Anspruch 6, **dadurch gekennzeichnet, daß** der Referenzwert bei 0,1 bis 2 % und vorzugsweise bei 0,4 bis 1 % liegt.

8. Analyseverfahren für die Diagnose eines Adenokarzinoms der Prostata bei einem männlichen menschlichen Patienten, ohne Vornahme einer Prostatabiospie, bei dem das in einer Blut-, Serum-, Urin- oder Samenflüssigkeitsprobe des Patienten vorhandene PSA(prostataspezifische Antigen)-Protein verwendet wird, **dadurch gekennzeichnet, daß** es besteht aus:

- dem Messen des Niveaus von komplexiertem PSA,

- dem Messen des Niveaus von gespaltenem freien PSA,

- dem Berechnen des Verhältnisses von gespaltenem freien PSA zu komplexiertem PSA, und

- dem Unterscheiden von zwei Fällen, nämlich:

  • dieses Verhältnis hat einen Wert, der größer oder gleich einem Referenz- oder Unterscheidungswert ist, der sich bei Patienten mit einem Adenokarzinom der Prostata ergibt, so daß der Patient von einem Adenokarzinom der Prostata betroffen ist,

  • dieses Verhältnis hat einen Wert, der kleiner als der Referenzwert ist, der sich bei Patienten mit gutartiger Pathologie der Prostata ergibt, so daß der Patient von einer gutartigen Pathologie der Prostata, wie z.B. einer gutartigen Prostatahyperthropie, betroffen ist.

9. Verfahren nach Anspruch 8, **dadurch gekennzeichnet, daß** der Referenzwert bei 0,1 bis 2,2 % und vorzugsweise bei 0,6 bis 1,2 % liegt.

10. Analyseverfahren für die Diagnose eines Adenokarzinoms der Prostata bei einem männlichen menschlichen Patienten, ohne Vornahme einer Prostatabiospie, bei dem das in einer Blut-, Serum-, Urin- oder Samenflüssigkeitsprobe des Patienten vorhandene PSA(prostataspezifische Antigen)-Protein verwendet wird, **dadurch gekennzeichnet, daß** es besteht aus:

- dem Messen des Niveaus von nicht-gespaltenem freien PSA,

- dem Messen des Niveaus von gespaltenem freien PSA,

- dem Messen des Niveaus von freiem PSA,

- dem Messen des Niveaus von gesamtem PSA,

- dem Berechnen des Verhältnisses von gespaltenem freien PSA zu nicht-gespaltenem freien PSA, das erstes Verhältnis genannt wird,

- dem Berechnen des Verhältnisses von freiem PSA zu gesamtem PSA, das zweites Verhältnis genannt wird, und

- dem Unterscheiden von vier Fällen, nämlich:

  • dieses erste Verhältnis hat einen Wert, der kleiner oder gleich einem ersten Referenz- oder Unterscheidungswert ist, und dieses zweite Verhältnis hat einen Wert, der kleiner oder gleich einem zweiten Referenz- oder Unterscheidungswert ist, so daß der Patient von einem Adenokarzinom der Prostata betroffen ist,

  • dieses erste Verhältnis hat einen Wert, der größer oder gleich einem ersten Referenz- oder Unterscheidungswert ist, und dieses zweite Verhältnis hat einen Wert, der größer oder gleich einem zweiten Referenz- oder Unterscheidungswert ist, so daß der Patient von einer gutartigen Pathologie der Prostata, wie z.B. einer Prostatahyperthropie, betroffen ist,

  • dieses erste Verhältnis hat einen Wert, der kleiner oder gleich einem ersten Referenz- oder Unterscheidungswert ist, und dieses zweite Verhältnis hat einen Wert, der größer oder gleich einem zweiten Referenz- oder Unterscheidungswert ist, so daß der Patient mit hoher Wahrscheinlichkeit von einem Adenokarzinom der Prostata betroffen ist, und mit geringer Wahrscheinlichkeit von einer gutartigen Pathologie der Prostata, wie z.B. einer gutartigen Prostatahyperthropie, betroffen ist,

  • dieses erste Verhältnis hat einen Wert, der größer oder gleich einem ersten Referenz- oder Unterscheidungswert ist, und dieses zweite Verhältnis hat einen Wert, der kleiner oder gleich einem zweiten Referenz- oder Unterscheidungswert ist, so daß der Patient mit hoher Wahrscheinlichkeit von einer gutartigen Pathologie der Prostata, wie z.B. einer gutartigen Prostatahyperthropie, betroffen ist, und mit einer geringen Wahrscheinlichkeit von einem Adenokarzinom der Prostata betroffen ist.

**11.** Verfahren nach Anspruch 10, **dadurch gekennzeichnet, daß** der erste Referenzwert bei 1 bis 18 %, vorzugsweise bei 5 bis 8 % liegt und genauer etwa 6,3 % beträgt, <u>und daß</u> der zweite Referenzwert bei 10 bis 20 %, vorzugsweise bei 13 bis 17 % liegt und genauer etwa 15 % beträgt.

**12.** Verfahren nach Anspruch 10 oder 11, **dadurch gekennzeichnet, daß** die hohe Wahrscheinlichkeit, von einem Adenokarzinom der Prostata betroffen zu sein, bei 80 bis 95 % und vorzugsweise bei 84 bis 87 %, und die geringe Wahrscheinlichkeit, von einer gutartigen Pathologie der Prostata betroffen zu sein, bei 5 bis 20 % und vorzugsweise bei 13 bis 16 % liegt, <u>und daß</u> die hohe Wahrscheinlichkeit, von einer gutartigen Pathologie der Prostata betroffen zu sein, bei 80 bis 95 % und vorzugsweise bei 82 bis 85 % liegt, und die geringe Wahrscheinlichkeit, von einem Adenokarzinom der Prostata betroffen zu sein, bei 5 bis 20 % und vorzugsweise bei 15 bis 18 % liegt.

**13.** Analyseverfahren für die Diagnose eines Adenokarzinoms oder einer gutartigen Pathologie der Prostata bei einem Patienten, bei dem die Kombination von zumindest zwei Verfahren verwendet wird, wobei jedes Verfahren nach einem der Ansprüche 1 oder 2 oder 3, oder auch 4 oder 5, oder auch 6 oder 7, oder auch 8 oder 9, oder auch 10 oder 11 oder 12 gebildet wird.

**14.** Verfahren nach einem der Ansprüche 1 bis 13, **dadurch gekennzeichnet, daß** es dann angewendet wird, wenn die GesamtPSA-Konzentration in dem Blut oder in dem Serum größer als 2 ng/ml ist.

**15.** Verfahren, in dem ein Verfahren nach einem der Ansprüche 1 bis 9 verwendet wird, **dadurch gekennzeichnet, daß** es besteht aus:

- dem Detektieren des Vorhandenseins und dem Quantifizieren des Niveaus von zwei der nachfolgenden Parameter: freies PSA in gespaltener Form, freies PSA in nicht-gespaltener Form, komplexiertes PSA und Gesamt-PSA,

- dem Bestimmen des Wertes des Verhältnisses von:

  • gespaltenem freien PSA zu gesamtem freien PSA,

  • gespaltenem freien PSA zu nicht-gespaltenem freien PSA,

  • nicht-gespaltenem freien PSA zu gesamtem freien PSA,

- gespaltenem freien PSA zu gesamten PSA, oder

- gespaltenem freien PSA zu komplexiertem PSA,

und

- dem Vergleichen dieses Verhältnisses mit einem Referenzwert für die Diagnose eines Adenokarzinoms der Prostata bei einem männlichen menschlichen Patienten.

**16.** Verfahren nach Anspruch 15, **dadurch gekennzeichnet, daß** man zuvor die Proteine, die aus dem Blut, dem Serum, dem Urin oder der Samenflüssigkeit des Patienten stammen, migrieren läßt.

**17.** Verfahren nach Anspruch 15 oder 16, **dadurch gekennzeichnet, daß** die Migration mittels zweidimensionaler Elektrophorese durchgeführt wird.

**18.** Verfahren nach einem der Ansprüche 15 bis 17, **dadurch gekennzeichnet, daß** zuvor der prozentuale Anteil des Gesamt-PSA bestimmt wird.

**19.** Verfahren nach einem der Ansprüche 15 oder 18, **dadurch gekennzeichnet, daß** die Detektion mittels Chemolumineszenz nach Darstellung mittels Westernblot durchgeführt wird.


**Claims**

**1.** Method of analysis for the purpose of the diagnosis of an adenocarcinoma of the prostate in a male human patient, without performing prostate biopsy, which uses the PSA (prostate specific antigen) protein present in a blood, serum, urine or seminal fluid sample from the patient, **characterized in that** it consists in:

- measuring the level of total free PSA, that is to say cleaved and noncleaved,
- measuring the level of cleaved free PSA,
- calculating the proportion of cleaved free PSA relative to the total free PSA, and
- distinguishing two cases, namely:

- this ratio has a value less than or equal to a reference or differentiating value, found in patients suffering from an adenocarcinoma of the prostate, such that the patient is suffering from an adenocarcinoma of the prostate,
- this ratio has a value greater than the reference value, found in patients suffering from a benign pathology of the prostate, such that the patient is suffering from a benign pathology of the prostate, such as a benign prostate hyperplasia.

**2.** Method of analysis for the purpose of the diagnosis of an adenocarcinoma of the prostate in a male human patient, without performing prostate biopsy, which uses the PSA (prostate specific antigen) protein present in a blood, serum, urine or seminal fluid sample from the patient, **characterized in that** it consists in:

- measuring the level of noncleaved free PSA,
- measuring the level of cleaved free PSA,
- calculating the proportion of cleaved free PSA relative to the noncleaved free PSA, and
- distinguishing two cases, namely:

- this ratio has a value less than or equal to a reference or differentiating value, found in patients suffering from an adenocarcinoma of the prostate, such that the patient is suffering from an adenocarcinoma of the prostate,
- this ratio has a value greater than the reference value, found in patients suffering from a benign pathology of the prostate, such that the patient is suffering from a benign pathology of the prostate, such as a benign prostate hyperplasia.

**3.** Method according to either of claims 1 and 2, **characterized in that** the reference value is between 2 and 12% and preferably between 5 and 8%.

4. Method of analysis for the purpose of the diagnosis of an adenocarcinoma of the prostate in a male human patient, without performing prostate biopsy, which uses the PSA (prostate specific antigen) protein present in a blood, serum, urine or seminal fluid sample from the patient, **characterized in that** it consists in:

- measuring the level of total free PSA, that is to say cleaved and noncleaved,
- measuring the level of noncleaved free PSA,
- calculating the proportion of noncleaved free PSA relative to the total free PSA, and
- distinguishing two cases, namely:

  • this ratio has a value greater than or equal to a reference or differentiating value, found in patients suffering from an adenocarcinoma of the prostate, such that the patient is suffering from an adenocarcinoma of the prostate,
  • this ratio has a value lower than the reference value, found in patients suffering from a benign pathology of the prostate, such that the patient is suffering from a benign pathology of the prostate, such as benign prostate hyperplasia.

5. Method according to claim 4, **characterized in that** the reference value is between 88 and 98% and preferably between 93 and 95%.

6. Method of analysis for the purpose of the diagnosis of an adenocarcinoma of the prostate in a male human patient, without performing prostate biopsy, which uses the PSA (prostate specific antigen) protein present in a blood, serum, urine or seminal fluid sample from the patient, **characterized in that** it consists in:

- measuring the level of total PSA, that is to say complexed and free, cleaved and noncleaved,
- measuring the level of cleaved free PSA,
- calculating the proportion of cleaved free PSA relative to the total PSA, and
- distinguishing two cases, namely:

  • this ratio has a value less than or equal to a reference or differentiating value, found in patients suffering from an adenocarcinoma of the prostate, such that the patient is suffering from an adenocarcinoma of the prostate,
  • this ratio has a value greater than the reference value, found in patients suffering from a benign pathology of the prostate, such that the patient is suffering from a benign pathology of the prostate, such as a benign prostate hyperplasia.

7. Method according to claim 6, **characterized in that** the reference value is between 0.1 and 2% and preferably between 0.4 and 1%.

8. Method of analysis for the purpose of the diagnosis of an adenocarcinoma of the prostate in a male human patient, without performing prostate biopsy, which uses the PSA (prostate specific antigen) protein present in a blood, serum, urine or seminal fluid sample from the patient, **characterized in that** it consists in:

- measuring the level of PSA complexed,
- measuring the level of cleaved free PSA,
- calculating the proportion of cleaved free PSA relative to the complexed PSA, and
- distinguishing two casns, namely:

  • this ratio has a value greater than or equal to a reference or differentiating value, found in patients suffering from an adenocarcinoma of the prostate, such that the patient is suffering from an adenocarcinoma of the prostate,
  • this ratio has a value less than the reference value, found in patients suffering from a benign pathology of the prostate, such that the patient is suffering from a benign pathology of the prostate, such as a benign prostate hyperplasia.

9. Method according to claim 8, **characterized in that** the reference value is between 0.1 and 2.2% and preferably between 0.6 and 1.2%.

10. Method of analysis for the purpose of the diagnosis of an adenocarcinoma of the prostate in a male human patient, without performing prostate biopsy, which uses the PSA (prostate specific antigen) protein present in a blood, serum, urine or seminal fluid sample from the patient, **<u>characterized in that</u>** it consists in:

- measuring the level of noncleaved free PSA,
- measuring the level of cleaved free PSA,
- measuring the level of free PSA,
- measuring the level of total PSA,
- calculating the proportion of cleaved free PSA relative to the noncleaved free PSA, called first ratio,
- calculating the proportion of free PSA relative to the total PSA, called second ratio, and
- distinguishing four cases, namely:

  - this first ratio has a value less than or equal to a first reference or differentiating value and this second ratio has a value less than or equal to a second reference or differentiating value, such that the patient is suffering from an adenocarcinoma of the prostate,
  - this first ratio has a value greater than or equal to a first reference or differentiating value and this second ratio has a value greater than or equal to a second reference or differentiating value, such that the patient is suffering from a benign pathology of the prostate, such as a benign prostate hyperplasia,
  - this first ratio has a value less than or equal to a first reference or differentiating value and this second ratio has a value greater than or equal to a second reference or differentiating value, such that the patient has a high probability of suffering from an adenocarcinoma of the prostate and a low probability of suffering from a benign pathology of the prostate, such as a benign prostate hyperplasia,
  - this first ratio has a value greater than or equal to a first reference or differentiating value and this second ratio has a value less than or equal to a second reference or differentiating value, such that the patient has a high probability of suffering from a benign pathology of the prostate, such as a benign prostate hyperplasia, and a low probability of suffering from an adenocarcinoma of the prostate.

11. Method according to claim 10, **<u>characterized in that</u>** the first reference value is between 1 and 18%, preferably between 5 and 8% and more precisely is substantially 6.3%, <u>and **in that**</u> the second reference value is between 10 and 20%, preferably between 13 and 17% and more precisely is substantially 15%.

12. Method according to either of claims 10 and 11, **<u>characterized in that</u>** the high probability of suffering from an adenocarcinoma of the prostate is between 80 and 95% and preferably between 84 and 87% and the low probability of suffering from a benign pathology of the prostate is between 5 and 20% and preferably between 13 and 16%, <u>and **in that**</u> the high probability of suffering from a benign pathology of the prostate is between 80 and 95% and preferably between 82 and 85%, and the low probability of suffering from an adenocarcinoma of the prostate is between 5 and 20% and preferably between 15 and 18%.

13. Method of analysis for the diagnosis of an adenocarcinoma or of a benign pathology of the prostate in a patient, which uses a combination of at least two methods, each method consisting of any one of claims 1 or 2 or 3, or alternatively 4 or 5, or alternatively 6 or 7, or alternatively 8 or 9, or alternatively 10 or 11 or 12.

14. Method according to any one of claims 1 to 13, **<u>characterized in that</u>** it is carried out when the total PSA concentration in the blood or in the serum is greater than 2 ng/ml.

15. Process using a method, according to any one of claims 1 to 14, **<u>characterized in that</u>** it consists in:

- detecting the presence and quantifying the level of two of the following parameters: free PSA in cleaved form, free PSA in noncleaved form, complexed PSA and total PSA,
- determining the value of the proportion of:

  - cleaved free PSA relative to the total free PSA,
  - cleaved free PSA relative to the noncleaved free PSA,
  - noncleaved free PSA relative to the total free PSA,
  - cleaved free PSA relative to the total PSA, or
  - cleaved free PSA relative to the complexed PSA,

  and
- comparing this proportion relative to a reference value, for the diagnosis of an adenocarcinoma of the prostate in a male human patient.

16. Process according to claim 15, **<u>characterized in that</u>** the proteins derived from the blood, serum, urine or seminal fluid from the patient are subjected to migration beforehand.

17. Process according to either of claims 15 and 16, **<u>characterized in that</u>** the migration is carried out by two-dimensional electrophoresis.

18. Process according to any one of claims 15 to 17, **<u>characterized in that</u>** the level of total PSA is assayed beforehand.

19. Process according to either of claims 15 and 18, **<u>characterized in that</u>** the detection is carried out by chemiluminescence after visualization by Western blotting.

Fig. 1

Fig. 2

Fig. 3

Fig. 4

Fig. 5

*Fig. 6*